# FASCICULE DE BREVET EUROPEEN

(11) **EP 3 113 738 B1**
(45) Date de publication et mention de la délivrance du brevet: **31.01.2018**
(21) Numéro de dépôt: 15707386.7
(22) Date de dépôt: 03.03.2015
(51) Int. Cl.: A61F 13/06, A61F 13/08, A61F 13/10

(54) **MANCHON DE COMPRESSION SANS COUTURES**
NAHTLOSE KOMPRESSIONSMANSCHETTE
SEAMLESS COMPRESSION SLEEVE

(30) Priorité: 03.03.2014 FR 1451673
(43) Date de publication de la demande: 11.01.2017
(73) Titulaire: Sigvaris AG, 9014 St. Gallen (CH)
(72) Inventeur: BAZOUD, Xavier, F-42610 Saint Romain le Puy (FR); DUHEM, Julien, 42600 Savigneux (FR)
(74) Mandataire: Cabinet Laurent & Charras
(86) Numéro de dépôt international: PCT/EP2015/054410
(87) Numéro de publication internationale: WO 2015/132256

(56) Documents cités:
- WO-A1-00/67600
- WO-A1-2006/015599
- DE-U1-202012 004 652
- JP-A- S 602 756
- US-A- 4 750 339
- US-B1- 6 230 524

## Description

### DOMAINE TECHNIQUE

La présente invention concerne une orthèse de compression pour le traitement du lymphoedème des membres supérieurs et plus particulièrement du bras. L'invention concerne plus particulièrement un manchon de compression réalisé intégralement sans coutures afin notamment d'améliorer le confort des patients portant le manchon de compression.

### ART ANTERIEUR

Le lymphoedème est un gonflement d'une partie plus ou moins importante du corps suite à une accumulation de liquide lymphatique dans les tissus conjonctifs. Le lymphoedème apparait lorsque les vaisseaux lymphatiques n'existent pas ou que les lymphangions qui le constituent sont dégradés ou non-fonctionnels. Le lymphoedème peut aussi se produire lorsque les vaisseaux lymphatiques sont endommagés, obstrués ou lorsque les nodules lymphatiques ont été enlevés suite à un dommage ou à un traumatisme causé par un accident, une chirurgie, une infection grave ou une radiothérapie. Outre le fait que le lymphoedème peut entraîner un handicap fonctionnel important et une altération marquée de la qualité de vie, il peut entrainer des complications telles que l'érysipèle qui est une infection de la peau due à une bactérie, le streptocoque.

Le traitement du lymphoedème consiste dans un premier temps à pratiquer un drainage lymphatique manuel dit DLM puis, dans un second temps, à porter une orthèse de compression telle qu'un bas de compression pour les membres inférieurs ou un manchon de compression pour les bras.

Le document WO00/67600 décrit un gant dont l'extrémité de la totalité des doigts, y compris celle du pouce est découpée. Ce gant présente des zones de différentes extensibilités qui sont reliées au reste du gant sans couture. Le gant est fabriqué dans sa totalité à partir, par exemple, d'un seul mélange de nylon et de lycra^{®}.

Le document WO2006/01559A1 décrit un produit orthopédique connu sous le nom de « spacer ». Cet article est réalisé sur un métier à chaine. Il associe deux couches externes reliées chacune par une couche intermédiaire. Dans un mode de réalisation particulier, il se présente sous la forme d'un tube recouvrant l'avant-bras et la main. Une ouverture est ménagée dans la paroi du tube pour le passage du pouce.

On connait également des manchons de compression destinés au traitement du lymphoedème du bras. Ils sont constitués d'un manchon tubulaire tricoté à partir de fils élastiques recouvrant au moins le bras et la main, au moins jusqu'à l'insertion des phalanges sur les os métacarpiens de la main, et un pouce ouvert. Un manchon de ce type est par exemple décrit dans le document DE202012004652U1.

Le document JP S60 2756A décrit un gant composé de fibres vinyles solubles dans leau, de fibres ayant la propriété de se contracter sous l'effet de la chaleur, et de fibres fusibles. Ces différents éléments, et notamment le chauffage des fils fusibles permettent de produire un gant tricoté qui ne se démaille pas aux extrémités.

Le document US 6 230 524, décrit un gant tricoté pour lequel les extrémités sont composées d'un fil de tricot enroulé au moins en partie sur un fil thermofusible présentant une température de fusion basse. Le chauffage permet d'éviter le démaillage des extrémités du gant.

Le document US 4 750 339 A décrit un article tricoté tel qu'un gant comportant une manchette de poignet comprenant une liaison de bord qui peut être activée par l'application de chaleur à celle-ci pour empêcher le décollement du bord de manchette. Le bord lié est défini par des première et seconde extrémités de fil qui sont tricotées ensemble, dans lequel le premier fil comprend un revêtement externe thermoplastique activable par chauffage et le second fil est défini par un matériau élastique non thermoplastique.

Le lymphoedème entrainant des modifications plus ou moins importantes du volume de la main et/ou des doigts, la réalisation des manchons de compression adaptés au traitement d'un patient nécessite un taillage des articles particulièrement précis. Pour ce faire, la technique actuelle consiste à obtenir le volume du pouce en utilisant un empiècement de compression tricoté indépendamment de la partie de compression principale et à utiliser une technique d'assemblage classique par confection afin de le solidariser au manchon tubulaire recouvrant le bras et le reste de la main. La solution d'assemblage de ces deux éléments la plus évidente et traditionnelle consiste en la réalisation d'une couture à la jonction du pouce et de la partie principale.

Ces deux parties, le manchon tubulaire et la pièce de pouce, étant réalisées initialement indépendamment l'une de l'autre, elles peuvent comporter des niveaux de compression différents. De plus, au niveau de la couture, la compression du produit n'est plus contrôlée puisque dans cette zone intervient un matériau externe aux caractéristiques mécaniques radicalement différentes de celles des empiècements de compression utilisés.

Par ailleurs, la zone constituée de la couture à la jonction du pouce et du manchon tubulaire génère très souvent une surépaisseur entrainant un inconfort liée à la superposition des deux parties. Cet inconfort est accentué car la zone de couture se trouve dans une zone de la main extrêmement mobile. Le niveau d'inconfort est propre à chaque patient ; néanmoins, ledit inconfort provient des frottements dus à la rigidité du fil de couture utilisé, la surépaisseur présente au niveau du tissu de compression et une certaine sensibilité cutanée liée à la pathologie considérée.

Enfin, le port d'un manchon de compression contre le lymphoedème de la main se révèle parfois gênant pour la réalisation des tâches quotidiennes. Le patient a parfois tendance dans ce cas à libérer sa main sans enlever complètement le manchon de compression en exerçant une traction vers l'avant dont le point d'appui se situe souvent à l'extrémité du pouce. Pour réussir à retirer le manchon de compression, il doit donc l'étirer fortement, ce qui provoque de très fortes tensions dans la mesure où celles-ci se doivent d'être supérieures au niveau de compression initiale pour pouvoir libérer le membre comprimé. Ces fortes contraintes mécaniques répétées parfois plusieurs fois par jour, finissent par fragiliser la jonction pouce/partie principale entrainant parfois des déchirures soit au niveau de la couture soit, ce qui est le plus fréquent, au niveau du tissu à proximité de la couture.

Par ailleurs, se pose le problème de la finition de la partie pouce dans la mesure où la partie terminale de celle-ci doit être ouverte pour permettre le passage dudit pouce. L'ouverture est effectuée par découpage au moyen d'un élément tranchant ce qui entraine inévitablement une fragilisation de la maille avec un risque de démaillage ultérieur.

### EXPOSE DE L'INVENTION

L'un des buts de l'invention est donc de remédier à ces inconvénients en proposant une orthèse de compression pour le traitement d'un lymphoedème de la main de conception simple et peu onéreuse, améliorant le confort au porter et la résistance à l'usage.

Un autre but est de mettre au point une orthèse ne présentant pas de risque de démaillage au niveau de l'extrémité distale du pouce après découpe.

A cet effet, et conformément à l'invention, il est proposé une orthèse de compression selon les revendications 1 à 6 comprenant un manchon tubulaire tricoté à partir de fils élastiques notamment, recouvrant au moins le bras et la main, au moins jusqu'à l'insertion des phalanges sur les os métacarpiens de la main, et une excroissance destinée à recouvrir au moins partiellement le pouce sur toute sa circonférence, le manchon tubulaire et le pouce étant tricotés d'un seul tenant.

L'orthèse est remarquable en ce que le pouce est tricoté sous la forme d'une pièce avantageusement globalement tronconique, au moins en partie à partir de fil(s) thermocollant(s) à une température T1, le manchon contenant éventuellement des fils thermocollants à une température T2 strictement supérieure à T1.

En pratique, les fils thermocollants contenus dans l'excroissance sont thermocollants à une température déterminée T1 de sorte que, après chauffage de l'orthèse à une température supérieure à la température de fusion desdits fils thermocollants à une température T1, ils collent entre eux. Dans ces conditions, après refroidissement, l'extrémité distale du pouce peut être découpée sans démaillage intempestif. Dans l'hypothèse où le manchon contient lui-même des fils thermocollants, ceux-ci sont thermocollants à une température supérieure strictement à T1 de sorte qu'à T1, ils conservent leur structure initiale et ne collent pas entre eux.

En pratique, la température T1 est comprise entre 100 et 130°C, de préférence entre 105 et 115°C, avantageusement de l'ordre de 110°C. Comme il sera vu par la suite, ces températures correspondent en pratique aux températures maximales mise en oeuvre pendant le procédé de fabrication de l'orthèse suivant les revendications 7 à 12. Par ailleurs, on comprend bien que contrairement aux orthèses de compression de l'art antérieur comprenant une couture reliant les pièces du pouce au manchon tubulaire, l'orthèse suivant l'invention ne comporte pas de couture de sorte que le confort du patient et la résistance à l'usage sont améliorés.

Avantageusement, le manchon et l'excroissance contiennent le même mélange de fibres textiles à l'exception du fil thermocollant à la température T1 présent uniquement dans l'excroissance. En pratique, les fibres textiles constitutives du manchon et de l'excroissance sont choisis dans le groupe comprenant le modal, la viscose, l'élasthanne, le polyamide. Dans un mode de réalisation avantageux, l'orthèse est tricotée à partir d'un mélange de modal, élasthanne et polyamide. Bien entendu, en fonction de zones du manchon, les caractéristiques des fibres peuvent varier, telles que par exemple leur diamètre. Lorsque l'orthèse contient des fils de polyamide en plus de fils thermocollants à la température T1, ces fils de polyamide sont thermocollants à une température T2 strictement supérieure à T1.

Selon un premier mode de réalisation, l'excroissance comporte deux parties non-symétriques tricotées d'un seul tenant, une première partie dite palmaire, s'étendant sur la face palmaire du pouce lorsque l'orthèse est portée, et une seconde partie dite dorsale, s'étendant sur la face dorsale du pouce lorsque l'orthèse est portée, présentant une largeur de la base supérieure à la largeur de la base de la partie palmaire et une hauteur supérieure à la hauteur de la partie palmaire.
De préférence, le rapport entre la largeur de la base de la partie palmaire du pouce et la largeur de l'orthèse est compris entre 0,25 et 0,35.
Par ailleurs, la base de la partie palmaire du pouce comporte entre 80 et 90 mailles, la base de la partie dorsale du pouce comporte entre 82 et 92 mailles, et l'extrémité distale avant découpe de la partie dorsale et de la partie palmaire du pouce comporte entre 26 et 36 mailles.
Selon un second mode de réalisation, l'excroissance comporte deux parties non-symétriques tricotées d'un seul tenant.

Le fil thermocollant à une température T1 consiste de préférence en un fil de copolyamide.
Enfin, pour permettre le passage du pouce au moment de sa mise en place, l'extrémité distale de l'excroissance est découpée.

Un autre objet de l'invention concerne un procédé de fabrication selon les revendications 7 à 12 d'une orthèse de compression selon les revendications 1 à 6 comprenant un manchon tubulaire tricoté à partir de fils élastiques notamment, recouvrant au moins le bras et la main, au moins jusqu'à l'insertion des phalanges sur les os métacarpiens de la main, et une excroissance recouvrant au moins partiellement le pouce sur toute sa circonférence, le manchon tubulaire et le pouce étant tricotés d'un seul tenant.

Le procédé selon les revendications 7 à 12 est remarquable en ce qu'il comporte au moins les étapes suivantes :
- tricotage d'un manchon tubulaire à partir de fils élastiques notamment sur une machine à tricoté circulaire,
- tricotage d'une excroissance avantageusement globalement tronconique à l'extrémité distale dudit manchon, ladite excroissance étant tricotée à partir au moins en partie de fil(s) thermocollant(s) à une température T1 et étant destinée à recouvrir au moins partiellement le pouce (2) sur toute sa circonférence,
- chauffage de l'orthèse à une température supérieure au point de fusion des fils thermocollants à une température T1 afin de fondre et coller lesdits fils thermocollants,
- refroidissement de l'orthèse.
L'étape de chauffage de l'orthèse consiste dans une étape de préfixation à une température T1. En pratique, la température T1 est comprise entre 100 et 130°C, de préférence entre 105 et 115°C, avantageusement de l'ordre de 110°C. La préfixation est conduite avantageusement pendant une durée comprise entre 1 et 2 minutes.

Le procédé comprend avantageusement une étape de teinture de l'orthèse entre l'étape de préfixation et l'étape de refroidissement. Cette étape est conduite à une température inférieure à T1, en pratique comprise entre 80 et 98°C, avantageusement de l'ordre de 95°C.
Selon une autre caractéristique le procédé contient en outre une étape de découpage de l'extrémité distale du pouce après refroidissement. En pratique, le découpage est effectué au moyen d'un outil tranchant.

Par ailleurs, l'étape de tricotage de l'excroissance formant le pouce consiste à tricoter d'un seul tenant deux parties symétriques ou non-symétriques.

Lorsque les parties sont non symétriques, l'étape de tricotage de l'excroissance formant le pouce consiste à tricoter d'un seul tenant, une première partie dite palmaire, s'étendant sur la face palmaire du pouce lorsque l'orthèse est portée, et une seconde partie dite dorsale, s'étendant sur la face dorsale du pouce lorsque l'orthèse est portée, présentant une largeur de la base supérieure à la largeur de la base de la partie palmaire et une hauteur supérieure à la hauteur de la partie palmaire.

De préférence, le rapport entre la largeur de la base de la partie palmaire du pouce et la largeur de l'orthèse est compris entre 0,25 et 0,35, la base de la partie palmaire du pouce est tricotée sur 80 à 90 aiguilles, et de préférence sur 85 aiguilles, la base de la partie dorsale du pouce est tricotée sur 82 à 92 aiguilles et, de préférence, sur 87 aiguilles, et l'extrémité proximale de la partie dorsale et de la partie palmaire du pouce est tricotée sur 26 à 36 aiguilles et, de préférence, sur 31 aiguilles.

De plus, le fil thermocollant à la température T1 consiste de préférence dans un fil de copolyamide tel que décrit précédemment.

### DESCRIPTION SOMMAIRE DES FIGURES

D'autres avantages et caractéristiques ressortiront mieux de la description qui va suivre, de plusieurs variantes d'exécution, données à titre d'exemples non limitatifs, de l'orthèse de compression pour le traitement d'un lymphoedème de la main suivant l'invention, à partir des dessins annexés sur lesquels :
- la figure 1 est une vue en perspective de l'orthèse de compression pour le traitement d'un lymphoedème de la main suivant l'invention,
- la figure 2 est une vue de dessus de l'orthèse de compression pour le traitement d'un lymphoedème de la main suivant l'invention portée par un patient,
- la figure 3 est une représentation schématique du patron de tricotage du pouce de l'orthèse de compression pour le traitement d'un lymphoedème de la main suivant l'invention,
- la figure 4 est une représentation graphique de la force à la rupture en fonction de la circonférence du pouce de plusieurs orthèses de compression pour le traitement d'un lymphoedème de la main suivant l'invention,
- la figure 5 est une représentation graphique de la force à la rupture en fonction de la circonférence du pouce de plusieurs orthèse de compression de l'art antérieur comprenant un pouce cousu au manchon tubulaire,
- la figure 6 est une représentation graphique de la force à la rupture moyenne en fonction de la circonférence du pouce d'orthèses de compression suivant l'invention et d'orthèses de compression de l'art antérieur.

### DESCRIPTION DETAILLEE DE L'INVENTION

Dans la suite de la description de l'orthèse de compression suivant l'invention pour le traitement d'un lymphoedème de la main, les mêmes références numériques désignent les mêmes éléments. Par ailleurs, les différentes vues ne sont pas nécessairement tracées à l'échelle.

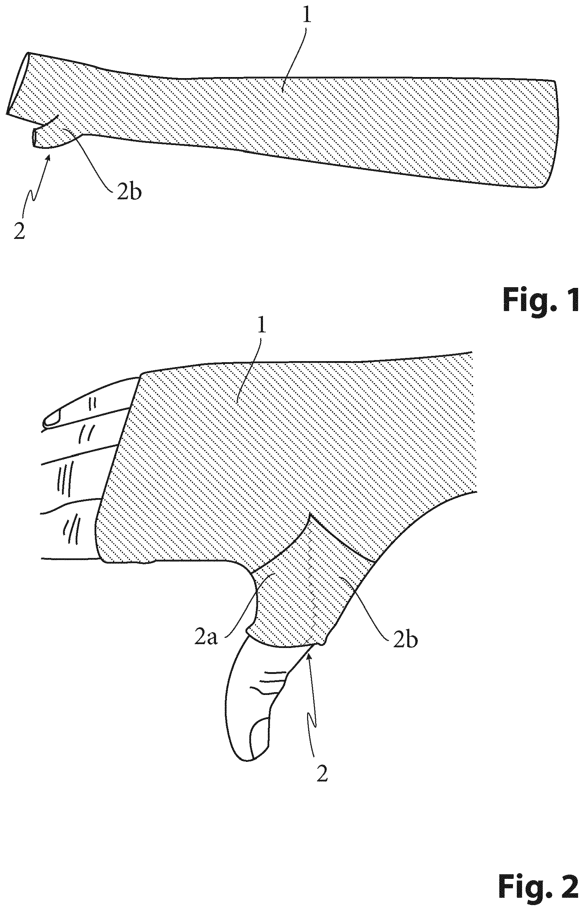

En référence aux figures 1 et 2, l'orthèse de compression suivant l'invention comprend un manchon tubulaire (1) tricoté à partir de fils élastiques recouvrant au moins le bras et la main, au moins jusqu'à l'insertion des phalanges sur les os métacarpiens de la main, et une excroissance destinée à recouvrir au moins partiellement et sur toute sa circonférence le pouce (2). Le manchon tubulaire (1) et le pouce (2) sont tricotés d'un seul tenant sur une machine à tricoter circulaire ou toute autre machine équivalente. Ledit pouce (2) est tricoté sous la forme d'une pièce globalement tronconique à partir au moins en partie de fils thermocollants à une température T1 déterminée de sorte que, après chauffage de l'orthèse à une température égale à T1, l'extrémité distale du pouce puisse être découpée sans démaillage intempestif après refroidissement. Ledit fil thermocollant consiste dans un fil de copolyamide dont le point de fusion est inférieur à 130°C. Ledit fil thermocollant peut, par exemple, consister dans un fil commercialisé par la société EMS-GRILTECH sous la référence GRILON (marque déposée) K-110.

Il est bien évident que le fil thermocollant pourra consister dans tout autre fil thermocollant bien connu de l'homme du métier sans pour autant sortir du cadre de l'invention.

Par ailleurs, l'homme du métier adaptera sans difficultés le diamètre du fil thermocollant en fonction de la texture du pouce recherchée notamment.

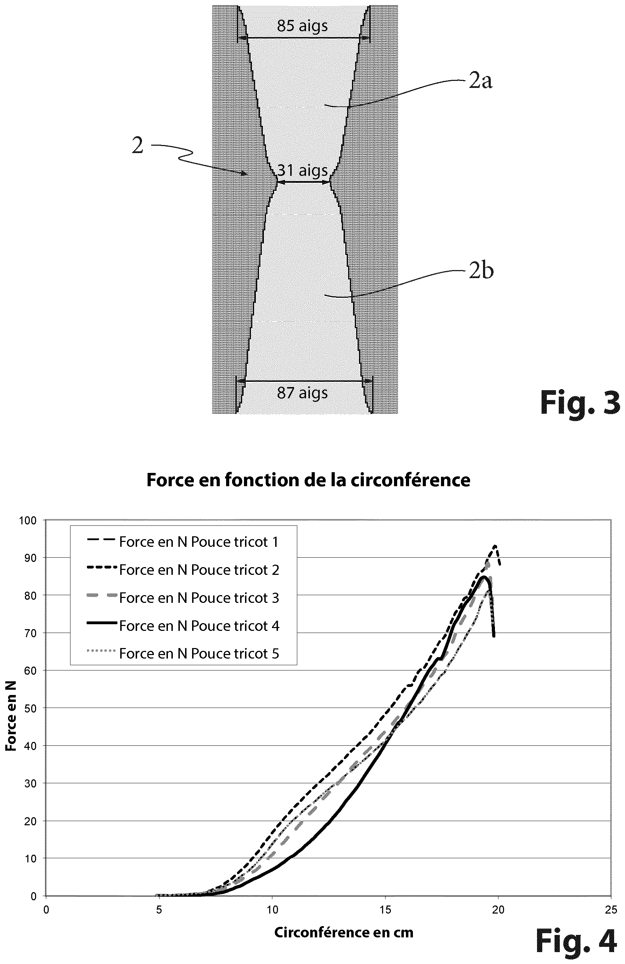

En référence aux figures 1 à 3, le pouce (2) comporte deux parties non-symétriques (2a,2b) tricotées d'un seul tenant, une première partie dite palmaire (2a), s'étendant sur la face palmaire du pouce lorsque l'orthèse est portée, et une seconde partie dite dorsale (2b), s'étendant sur la face dorsale du pouce lorsque l'orthèse est portée. La seconde partie dorsale (2b) présente une largeur de la base supérieure à la largeur de la base de la partie palmaire (2a) et une hauteur supérieure à la hauteur de la partie palmaire (2a). Le rapport entre la largeur de la base de la partie palmaire (2a) du pouce (2) et la largeur de l'orthèse est de préférence compris entre 0,25 et 0,35. La base de la partie palmaire (2a) du pouce (2) comporte entre 80 et 90 mailles et la base de la partie dorsale (2b) du pouce (2) comporte entre 82 et 92 mailles, en référence à la figure 3, le manchon tubulaire (1) comportant environ 290 mailles. Par ailleurs, l'extrémité distale de la partie dorsale (2b) et de la partie palmaire (2a) du pouce comporte entre 26 et 36 mailles. De plus la hauteur de la partie dorsale (2b) est supérieure à la hauteur de la partie palmaire (2a) du pouce (2), la partie dorsale (2b) présentant de préférence une hauteur comprise entre 140 et 160 rangées de maille et la partie palmaire (2a) présentant de préférence une hauteur comprise entre 110 et 120 rangées de maille.

Accessoirement, la partie palmaire (2a) et/ou la partie dorsale (2b) pourra comprendre des lignes transversales, imprimées ou réalisées au moyen de fils de couleurs, formant un ou plusieurs repères pour qu'un patient puisse découper, et in fine ouvrir, l'extrémité distale du pouce (2).

On observera que l'extrémité distale du pouce (2) pourra également être prédécoupée sans pour autant sortir du cadre de l'invention. En pratique, le découpage du pouce peut être directement effectué soit par le fabriquant de l'orthèse, soit par le patient, sous le contrôle de son médecin. L'avantage dans ce dernier cas est que le patient peut exactement adapter la taille du pouce.

Selon l'invention, le reste de l'orthèse, à savoir le manchon et le reste du pouce est tricoté à partir d'un mélange de de modal, élasthanne et polyamide thermocollant à une température T2 strictement supérieure à T1. Par conséquent, il n'y a pas de risque de collage des fils entre eux au moment du thermocollage des fils constitutifs du pouce, c'est-à-dire au moment de l'étape de préfixation.

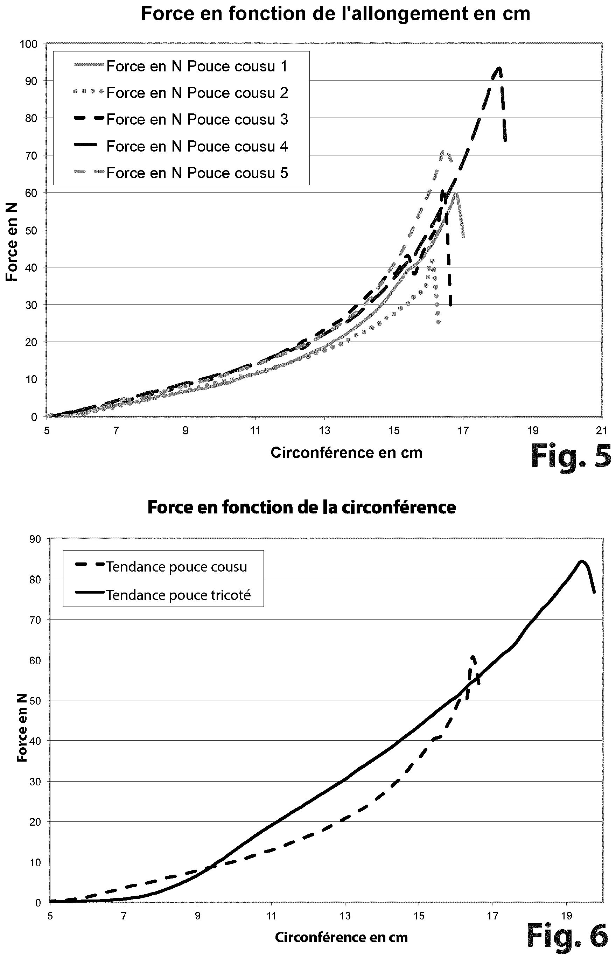

On notera que compte-tenu de l'absence de couture entre le pouce (2) et le manchon tubulaire (1), le patient portant l'orthèse suivant l'invention ne sera pas gêné par la surépaisseur de la couture notamment. De, plus en référence aux figures 4 à 6, il apparaît clairement que la force à la rupture des orthèses suivant l'invention, ne comportant pas de couture, est supérieure à la force de rupture des orthèses de l'art antérieur comportant une couture. En effet, on peut constater, en référence à la figure 6, que la force à la rupture des orthèses suivant l'invention est supérieure d'environ 16% à la force de rupture des orthèses de l'art antérieur. Les mesures de la force à la rupture ont été réalisées au moyen d'un dynamomètre, la force à la rupture étant la force à laquelle on mesure une chute de plus de 20% par rapport à la valeur de la force précédemment mesurée.
On décrira ci-après le procédé de fabrication de l'orthèse de compression suivant l'invention. Ledit procédé comporte au moins les étapes suivantes de tricotage d'un manchon tubulaire (1) à partir de fils élastiques notamment, sur une machine à tricoter circulaire, de tricotage d'une excroissance globalement conique à l'extrémité distale dudit manchon (1), ladite excroissance formant le pouce (2) de l'orthèse, à partir d'au moins un fils thermocollants à une température T1, et de chauffage de l'orthèse à une température supérieure au point de fusion des fils thermocollants à une température T1 afin de fondre et coller lesdits fils thermocollants.
De préférence, l'étape de chauffage de l'orthèse pour faire fondre le fil thermocollant consiste dans une étape de préfixation à une température T1. Dans cet exemple particulier de réalisation, le fil thermocollant à une température T1 consiste dans un fil de copolyamide dont le point de fusion est d'environ 125°C, commercialisé par la société EMS-GRILTECH sous la référence GRILON (marque déposée) K-110 ; toutefois, il est bien évident que le fil thermocollant pourra consister dans tout fil thermocollant bien connu de l'homme du métier et dont le point de fusion est de préférence inférieur ou égal à 130°C.
L'étape de tricotage de l'excroissance formant le pouce consiste à tricoter d'un seul tenant deux parties non-symétriques, une première partie dite palmaire (2a), s'étendant sur la face palmaire du pouce lorsque l'orthèse est portée, et une seconde partie dite dorsale (2b), s'étendant sur la face dorsale du pouce lorsque l'orthèse est portée, présentant une largeur de la base supérieure à la largeur de la base de la partie palmaire (2a) et une hauteur supérieure à la hauteur de la partie palmaire (2a). Le rapport entre la largeur de la base de la partie palmaire du pouce et la largeur de l'orthèse est compris entre 0,25 et 0,35. La base de la partie palmaire (2a) du pouce (2) est tricotée sur 80 à 90 aiguilles, et de préférence sur 85 aiguilles, et la base de la partie dorsale (2b) du pouce (2) est tricotée sur 82 à 92 aiguilles et, de préférence, sur 87 aiguilles. L'extrémité proximale de la partie dorsale (2b) et de la partie palmaire (2a) du pouce (2) est tricotée sur 26 à 36 aiguilles et, de préférence, sur 31 aiguilles.

## Revendications

1. Orthèse de compression comprenant un manchon tubulaire (1) tricoté à partir de fils élastiques notamment, recouvrant au moins le bras et la main, au moins jusqu'à l'insertion des phalanges sur les os métacarpiens de la main, et une excroissance destinée à recouvrir au moins partiellement le pouce (2) sur toute sa circonférence, le manchon tubulaire (1) et l'excroissance (2) étant tricotés d'un seul tenant, ***caractérisée* en ce que** l'excroissance (2) est tricotée au moins en partie à partir de fil(s) thermocollant(s) à une température T1 ; ladite température T1 est comprise entre 100 et 130°C et ; lesdits manchon tubulaire (1) et excroissance (2) contiennent le même mélange de fibres textiles à l'exception du fil thermocollant à la température T1 présent uniquement dans l'excroissance (2).

2. Orthèse de compression selon la revendication précédente, **caractérisée en ce que** le manchon contient des fils thermocollants à une température T2 strictement supérieure à T1.

3. Orthèse de compression suivant l'une des revendications précédentes, **caractérisée en ce que** la température T1 est comprise entre 105 et 115°C, avantageusement de l'ordre de 110°C.

4. Orthèse de compression suivant l'une des revendications précédentes, **caractérisée en ce que** les fibres textiles constitutives du manchon et de l'excroissance sont choisis dans le groupe comprenant le modal, la viscose, l'élasthanne, le polyamide.

5. Orthèse de compression suivant l'une des revendications précédentes, **caractérisée en ce que** le fil thermocollant à T1 consiste en un fil de copolyamide.

6. Orthèse selon l'une des revendications précédentes, **caractérisée en ce que** l'extrémité distale de l'excroissance est découpée.

7. Procédé de fabrication d'une orthèse de compression selon l'une des revendications 1 à 6, **caractérisé en ce qu'**il comporte au moins les étapes suivantes de :
- tricotage d'un manchon tubulaire à partir de fils élastiques notamment, sur une machine à tricoter circulaire,
- tricotage d'une excroissance à l'extrémité distale dudit manchon, ladite excroissance étant tricotée à partir, au moins en partie, de fil(s) thermocollant(s) à une température T1 et étant destinée à recouvrir au moins partiellement le pouce (2) sur toute sa circonférence,
- chauffage de l'orthèse à une température supérieure au point de fusion des fils thermocollants à une température T1 afin de fondre et coller lesdits fils thermocollants,
- refroidissement de l'orthèse.

8. Procédé selon la revendication 7, caractérisé en ce l'étape de chauffage consiste dans une étape de préfixation à une température T1.

9. Procédé selon l'une des revendications 7 ou 8, **caractérisé en ce qu'**il comprend une étape de teinture entre l'étape de préfixation et l'étape de refroidissement.

10. Procédé selon la revendication 9, **caractérisé en ce que** l'étape de teinture est conduite à une température inférieure à T1.

11. Procédé selon la revendication 9, **caractérisé en ce que** la température est comprise entre 80 et 98°C, avantageusement de l'ordre de 95°C.

12. Procédé selon l'une des revendications 7 à 11, **caractérisé en ce qu'**il comprend en outre une étape de découpage de l'extrémité distale du pouce.

## Patentansprüche

1. Kompressionsorthese, umfassend eine schlauchförmige Hülse (1), die insbesondere aus elastischen Fäden gestrickt ist und zumindest den Arm und die Hand zumindest bis zum Ansatz der Fingerglieder an den Mittelhandknochen der Hand bedeckt, und eine Ausstülpung, die dazu bestimmt ist, den Daumen (2) an seinem gesamten Umfang zumindest teilweise zu umhüllen, wobei die schlauchförmige Hülse (1) und die Ausstülpung (2) in einem Stück gestrickt sind, **dadurch gekennzeichnet, dass** die Ausstülpung (2) zumindest zum Teil aus einem wärmeklebenden Faden (wärmeklebenden Fäden) bei einer Temperatur T1 gestrickt ist; wobei die Temperatur T1 im Bereich zwischen 100 und 130°C liegt; wobei die schlauchförmige Hülse (1) und die Ausstülpung (2) das gleiche Gemisch von Textilfasern enthalten, mit Ausnahme des wärmeklebenden Fadens bei der Temperatur T1, der allein in der Ausstülpung (2) vorhanden ist.

2. Kompressionsorthese gemäß dem vorhergehenden Anspruch, **dadurch gekennzeichnet, dass** die Hülse wärmeklebende Fäden bei einer Temperatur T2 enthält, die strikt über T1 liegt.

3. Kompressionsorthese gemäß einem der vorhergehenden Ansprüche, **dadurch gekennzeichnet, dass** die Temperatur T1 im Bereich zwischen 105 und 115°C, vorteilhafterweise in der Größenordnung von 110°C liegt.

4. Kompressionsorthese gemäß einem der vorhergehenden Ansprüche, **dadurch gekennzeichnet, dass** die Textilfasern, welche die Hülse und die Ausstülpung bilden, aus der Gruppe, bestehend aus Modal, Viskose, Elasthan, Polyamid, ausgewählt sind.

5. Kompressionsorthese gemäß einem der vorhergehenden Ansprüche, **dadurch gekennzeichnet, dass** der wärmeklebende Faden bei T1 aus einem Copolyamidfaden besteht.

6. Orthese gemäß einem der vorhergehenden Ansprüche, **dadurch gekennzeichnet, dass** das distale Ende der Ausstülpung aufgeschnitten ist.

7. Verfahren zur Herstellung einer Kompressionsorthese gemäß einem der Ansprüche 1 bis 6, **dadurch gekennzeichnet, dass** es zumindest die folgenden Schritte umfasst:
- Stricken einer schlauchförmigen Hülse insbesondere aus elastischen Fäden auf einer Rundstrickmaschine,
- Stricken einer Ausstülpung am distalen Ende der Hülse, wobei die Ausstülpung zumindest zum Teil aus einem wärmeklebenden Faden (wärmeklebenden Fäden) bei einer Temperatur T1 gestrickt wird und dazu bestimmt ist, den Daumen (2) an seinem gesamten Umfang zumindest teilweise zu umhüllen,
- Erwärmen der Orthese auf eine Temperatur oberhalb des Schmelzpunkts der wärmeklebenden Fäden bei einer Temperatur T1, um die wärmeklebenden Fäden aufzuschmelzen und aneinanderzukleben,
- Abkühlen der Orthese.

8. Verfahren gemäß Anspruch 7, **dadurch gekennzeichnet, dass** der Erwärmungsschritt in einem Vorfixierungsschritt bei einer Temperatur T1 besteht.

9. Verfahren gemäß einem der Ansprüche 7 oder 8, **dadurch gekennzeichnet, dass** es einen Färbeschritt zwischen dem Vorfixierungsschritt und dem Abkühlungsschritt umfasst.

10. Verfahren gemäß Anspruch 9, **dadurch gekennzeichnet, dass** der Färbeschritt bei einer Temperatur unterhalb von T1 durchgeführt wird.

11. Verfahren gemäß Anspruch 9, **dadurch gekennzeichnet, dass** die Temperatur im Bereich zwischen 80 und 98°C, vorteilhafterweise in der Größenordnung von 95°C liegt.

12. Verfahren gemäß einem der Ansprüche 7 bis 11, **dadurch gekennzeichnet, dass** es ferner einen Schritt des Aufschneidens des distalen Daumenendes umfasst.

## Claims

1. Compression orthosis comprising a tubular sleeve (1) in particular knit from elastic yarn, covering at least the arm and the hand, at least up to the insertion of the phalanges on the metacarpal bones of the hand, and a protrusion intended to at least partially cover the thumb (2) over its entire circumference, the tubular sleeve (1) and the protrusion (2) being knit into one piece, **characterised in that** the protrusion (2) is knit at least in part from the yarn(s) thermobonding at a T1 temperature; said T1 temperature being comprised between 100 and 130°C and ; said tubular sleeve (1) and protrusion (2) containing the same combination of textile fibres with exception of the yarn thermobonding at the T1 temperature that is present in the protrusion (2) only.

2. Compression orthosis according to the preceding claim, **characterised in that** the sleeve contains yarn thermobonding at a T2 temperature strictly higher than T1.

3. Compression orthosis according to one of the preceding claims, **characterised in that** the T1 temperature is comprised between 105 C and 115 °C, more preferably, around 110 °C.

4. Compression orthosis according to one of the preceding claims, **characterised in that** the textile fibres constituting the sleeve and the projection are selected from the group comprising modal, viscose, elastane, and polyamide.

5. Compression orthosis according to one of the preceding claims, **characterised in that** the yearn thermobonding at T1 is a copolyamide yearn.

6. Compression orthosis according to one of the preceding claims, **characterised in that** the distal end of the protrusion is cut off.

7. Manufacturing process of a compression orthosis according to one of claims 1 to 6, **characterised in that** it includes at least the following stages of:
- knitting a tubular sleeve in particular from elastic thread, on a circular knitting machine,
- knitting a protrusion at the distal end of said sleeve, said protrusion being knit from, at least in part, thermobonding yarn(s) at a T1 temperature and being destined to at least partially cover the thumb (2) over its entire circumference,
- heating the orthosis to a temperature above melting point of the yarns thermobonding at a T1 temperature so as to melt and stick together the said thermobonding yarns,
- cooling the orthosis.

8. Manufacturing process according to claim 7, **characterised in that** the heating stage consists of a pre-fixing stage at a T1 temperature.

9. Manufacturing process according to one of claims 7 or 8, **characterised in that** it includes a dyeing stage between the pre-fixing stage and the cooling stage.

10. Manufacturing process according to claim 9, **characterised in that** the dyeing stage is performed at a temperature lower than T1.

11. Manufacturing process according to claim 9, **characterised in that** the temperature is between 80 and 98 °C, preferably about 95 °C.

12. Manufacturing process according to one of claims 7 to 11, **characterised in that** it further includes a cutting stage of the distal end of the thumb.
